Europäisches Patentamt

European Patent Office

Office européen des brevets

(19)

(11) Publication number: **0 284 849 B1**

## EUROPEAN PATENT SPECIFICATION

(12)

(45) Date of publication of patent specification: **04.08.93**　(51) Int. Cl.⁵: **A61K 9/22**, A61K 9/26

(21) Application number: **88103715.4**

(22) Date of filing: **09.03.88**

(54) **Sustained release tablets on the basis of high molecular weight hydroxypropylmethylcellulose and a process for their manufacture.**

(30) Priority: **11.03.87 YU 406/87**

(43) Date of publication of application:
**05.10.88 Bulletin　88/40**

(45) Publication of the grant of the patent:
**04.08.93 Bulletin　93/31**

(84) Designated Contracting States:
**BE CH DE FR GB LI**

(56) References cited:
**EP-A- 0 156 592**
**GB-A- 2 170 407**
**GB-A- 2 181 053**

(73) Proprietor: **LEK, tovarna farmacevtskih in kemicnih izdelkov, n.sol.o.**
**Verovskova 57**
**SI-61107 Ljubljana(SI)**

(72) Inventor: **Mohar, Milojka**
**Chvatalova 9**
**SI-61230 Domzale(SI)**
Inventor: **Kremzar, Ljuba**
**Prusnikova 38**
**SI-61210 Ljubljana-Sentvid(SI)**
Inventor: **Jerala-Strukelj, Zdenka**

**Podreca 107**
**SI-64211 Mavcice(SI)**
Inventor: **Urbancic-Smerkolj, Janja**
**Titova 21**
**SI-61000 Ljubljana(SI)**
Inventor: **Lenardic, Andrej**
**Mackov kot 7**
**SI-61000 Ljubljana(SI)**
Inventor: **Cvelbar, Polona**
**Velike Lasce 14**
**SI-61315 Velike Lasce(SI)**
Inventor: **Lah-Gros, Frida**
**Kvedrova 3**
**SI-61000 Ljubljana(SI)**
Inventor: **Nikolic, Vida**
**Ob zici 3**
**SI-61000 Ljubljana(SI)**
Inventor: **Lampret, Marija**
**Sentvid pri Sticni 23**
**SI-61296 Sentvid pri Sticni(SI)**
Inventor: **Mavec, Mihela**
**Klanec 35**
**SI-61291 Skofljica(SI)**

Rank Xerox (UK) Business Services
(3.10/3.6/3.3.1)

EP 0 284 849 B1

Inventor: **Zorz, Mirjan**
**Presernova 53**
**SI-61290 Grosuplje(SI)**
Inventor: **Kopitar, Zdravko**
**Muljava 7**
**SI-61234 Menges(SI)**


(74) Representative: **Müller-Boré & Partner Paten-**
**tanwälte**
**Isartorplatz 6 Postfach 26 02 47**
**W-8000 München 2 (DE)**

## Description

Technical Field of the Invention

The present invention relates to sustained release tablets (retard tablets) for oral administration on the basis of high molecular weight hydroxypropylmethylcellulose.

More particularly,,the invention relates to tablets having controlled and sustained activity comprising aminophylline, theophylline, Ketoprofen and propanolol hydrochloride as therapeutically active agents on a carrier base material comprising a hydroxypropylmethylcellulose having a defined chemical structure and molecular weight, a microcrystalline cellulose, glycerol ditripalmitostearate acting as a lubricant with retarding activity, together with other conventional pharmaceutically acceptable adjuvants. More specifically, the carrier is a high viscosity grade hydroxypropylmethyl-cellulose having an average molecular weight of more than 50 000, a 28-30 weight-% methoxyl content and a 7.5 to 12 weight-% hydroxypropoxyl content, known as Methocel E4M Premium.

Technical Problem

There exists a constant need for orally administered solid drug unit dosage forms having a controlled and prolonged activity, wherein the therapeutic agent is released from the medicament either intermittently at fixed intervals or continuously over a longer period.

Prior Art

The prolonged activity is achieved by controlled dissolution and/or diffusion of the therapeutic agent from the dosage form. To this end, various carrier base materials are used, such as waxes, fatty substances, polymers, naturally occuring, semisynthetic or synthetic materials etc. Among hydrophilic substances, hydroxypropylmethylcellulose is an important type of such substances due to its pH-independent activity as well as its synthetic origin. The use of hydroxypropylmethylcelluloses as carrier base materials having a defined chemical structure and molecular weight in sustained release tablets is well-known.

These water-soluble or hydrophilic cellulose ethers are commercially available in various viscosity grades under several trade names. The various grades available under a given trade name represent differences in composition as well as in molecular weight.

Water-soluble methylcellulose (Methocel A, previously designated as Methocel MC,from The Dow Chemical Co., U. S. A., and Metalose SM, from Shin-Etsu, Ltd., Japan) has a methoxyl content of 27.5 to 31.5 weight-% and is available in various viscosity grades. Hydroxypropylmethylcellulose is essentially a series of compounds (Methocel E, F, J and K, all previously designated as versions of Methocel HG, from The Dow Chemical Co., and of Metalose SH, from Shin-Etsu, Ltd., Japan) having each a different chemical composition with methoxyl content ranging from 16.5 to 30 weight-% and hydroxypropoxyl content ranging from 4 to 32 weight-%, each of them being available in various viscosity grades.

Commercial designations of various cellulose ethers are based on the viscosities of 2 % aqueous solutions at 20° C. The viscosities range from $5x10^{-3}$ Pa.s. to 100 Pa.s. and represent average molecular weights ranging from 10 000 to over 150 000 as calculated from the data in "Handbook of Methocel Cellulose Ether Products" (The Dow Chemical Co., 1974)

Christenson and Dale (U.S. patent 3,065,143) and Huber, Dale and Christenson (J. Pharm. Sci. 55, 974 (1966)) disclosed sustained release tablets, containing at least one third part by weight of the tablet of a high viscosity grade hydroxypropylmethylcellulose as binder.

As binders, Methocel 60 HG 4 Pa.s (4000 cps), now known as Methocel E4M, having a 28-30 weight-% methoxyl content and a 7.5 to 12 weight-% hydroxypropoxyl content and an average molecular weight of 93 000 as well as Methocel 90 HG 4 Pa.s (4000 cps) and Methocel 90 HG 15 Pa.s (15 000 cps), now known as Methocel K4M and Methocel K15M, respectively, having a 19-29 weight-% methoxyl content, a 4-12 weight-% hydroxypropoxyl content and an average molecular weight of 89 000 and 124 000, respectively, were used.

Christenson et al. assumed that water was rapidly absorbed and that a gel barrier was formed on the surface of the tablet. The release of the therapeutic agent was controlled by the diffusion of the therapeutic agent across the gel barrier.

Christenson and Huber (U.S. patent 3,590,117) reported that neither low not high viscosity grade hydroxypropylmethylcellulose made acceptable long-lasting troches.

Lapidus (Dissertation, Rutgers State University, 1967) and Lapidus and Lordi (J. Pharm. Sci. 55, 840 (1966); 57, 1292 (1968)) studied the use of high viscosity grade methylcellulose 4 Pa.s (4000 cps), now designated as Methocel A4M, and/or low and high viscosity grade hydroxypropylmethylcellulose $2.5 \times 10^{-2}$ Pa.s (25 cps) and 15 Pa.s (15 000 cps) viscosity grade Methocel 90HG, now designated as Methocel K25 and Methocel K15M, respectively) in solid drug unit dosage forms and confirmed the assumption of Christenson et al. that the rate of therapeutic agent release across the gel barrier was determined by the therapeutic agent diffusion across the gel barrier and by the attrition of the latter.

Salomon, Doelker and Buri (Pharm. Acta Helv. 54 (3), 82 (1979) disclosed the use of 15 000 cps viscosity grade Methocel 90HG (now designated as Methocel K 15M) in tablets containing potassium chloride.

Sheth and Tossounian (U.S. patents 4,126,672, 4,140,755 and 4,167,558) disclosed solid drug unit dosage forms containing 4 Pa.s (4000 cps) viscosity grade methylcellulose or hydroxypropylmethylcellulose in combination with various additives including gas-generating compounds, e.g. calcum carbonate, and inert materials so as to be hydrodynamically balanced so that they have a bulk density of less than one.

Schor, Nigalaye and Gaylord (U.S. patent 4,389,393) disclosed sustained release solid drug unit dosage forms in which the carrier constituted less than one third, more particularly less than 25.8 weight-%, of the weight of the tablet, and consisted of hydroxypropylmethylcellulose, having a methoxyl content of 16 to 24 weight-%, a hydroxypropoxyl content of 4-32 weight-% and an average molecular weight of at least 50 000, i.e. Methocel J and Methocel K or Metalose 90SH.

The use of high viscosity grades of methylcellulose Methocel A and hydroxypropylmethylcellulose Methocel E, Methocel F and Methocel K in sustained release solid drug unit dosage forms is also described in the technical bulletin "Formulating Sustained Release Pharmaceutical Products with Methocel" (The Dow Chemical Co., 1982).

The cited prior art discloses that high viscosity grades of hydroxypropylmethylcellulose of various chemical compositions are useful in the preparation of sustained relase solid drug unit dosage forms.

Schor, Nigalaye and Gaylord (U.S. patent 4,369,172) disclosed effective prolonged release therapeutic compositions, prepared by using a low viscosity grade hydroxypropylmethylcellulose having a hydroxypropoxyl content of 9-12 weight-% and an average molecular weight of less than 50 000 as the carrier base material.

Lowey and Stafford (U.S. Patent 3,870,790) and Schor (U.S. patent 4,226,489) disclosed that sustained release tablets were produced by using as the carrier base material a modified low viscosity grade hydroxypropylmethylcellulose having a hydroxypropoxyl content of less than 9 weight-% and an average molecular weight of 23 000, e.g. Methocel E50.

Lowey (U.S. patent 4,259,314) disclosed the use of a mixture of hydroxypropylmethylcellulose having a viscosity of $5 \times 10^{-2}$ to 4 Pa.s., and hydroxypropylcellulose as the carrier base material in the preparation of sustained release pharmaceutical compositions.

Gaylord and Schor (EP-A-0 157 695) disclosed a sustained release solid drug unit dosage form comprising a carrier base material and a therapeutic agent, the carrier base material being a mixture of one or more non-ionic cellulose ethers and an anionic surfactant, wherein at least one cellulose ether is methylcellulose or hydroxypropylmethylcellulose having an average molecular weight of at least 50 000.

Davis and Gaylord (U.S. patent 4,540,566) disclosed a formulation similar to that disclosed in said European Patent Application, the carrier base material, however, being modified hydroxypropylmethylcellulose having an average molecular weight of less than 50 000.

U.K. patent application GB-A-2 170 407 relates to controlled release galenic forms (capsules) for oral administration, comprising a 9,10-dihydro ergot alkaloid (co-dergocrine, dihydroergotamine, epicryptine or 9'-thia-9,10-dihydroergotamine) as a drug, a pharmaceutically acceptable hydrophilic swelling substance (preferably hydroxypropylmethylcellulose) and a pharmaceutically acceptable inert fatty material (preferably cetyl palmitate). The bioavailability of these retarded formulations in standard clinical trials can be compared to that of conventional non-retarded formulations containing the same amounts of alkaloids.

European patent application EP-A-0 156 592 concerns sustained release pharmaceutical dosage forms comprising a hard gelatin capsule containing oxazepam, ciramadol, guanabenz and lorazepam as the drugs together with Methocel K type hydroxypropylmethylcellulose (in the application referred to as USP 2208) as the carrier. The capsules are prepared avoiding the expensive compression and granulation processing by simply blending a mixture of the ingredients and filling hard gelatin capsule therewith without loss of the sustained release effect observed in compressed tablets.

Description of the Solution of the Technical Problem with Examples of Embodiment

The present invention relates to sustained release tablets or retard tablets comprising aminophylline, theophylline, ketoprofen and propanolol hydrochloride as therapeutically active agents. The carrier base material for manufacturing the tablets is high viscosity grade hydropropylmethylcellulose having an average molecular weight of over 50 000, a 28-30 weight-% methoxyl content and a 7.5-12 weight-% hydroxypropoxyl content, i.e. the commercially available Methocel 60HG 4 Pa.s (4000 cps), now designated as Methocel E4M, which represents from 20 to 40 weight-% of the weight of the tablet. The tablets additionally contain up to 10 weight-% of glycerol ditripalmitostearate (Precirol AT05) as a pharmaceutically acceptable inert fatty substance acting as a lubricant with retarding action as well as from 10 to 20 weight-% of microcrystalline cellulose (Avicel$^R$) and other pharmaceutically acceptable adjuvants. In the cited prior art literature no mention is made of the use of Methocel E4M 4 Pa.s (4000 cps) in the proposed formulations with microcrystalline cellulose and glycerol ditripalmitostearate for hygroscopic and also for non-hygroscopic therapeutic agents. Said combination is particularly suitable for the preparation of sustained release systems of the present invention having advantageous characteristics of the active agent release as demonstrated in the examples and showing a bioavailability in clinical tests that is comparable with or superior to that of known commercial preparations. No prior chemical or physical treatment of hydroxypropylmethylcellulose as proposed in some instances in the cited prior art is necessary for manufacturing the tablets of the present invention. Upon addition of the therapeutic agent and other ingredients, the mixture has an excellent compressibility and the tablets prepared therefrom are hard, stable and of low friability and provide a slow release rate of the therapeutic agent.

The tableting of some therapeutic agents can be achieved by direct compressing without prior granulation, e.g. in the manufacture of aminophylline and propranolol hydrochloride sustained release tablets. For the manufacture of sustained release tablets of the present invention there can be used aminophylline, theophylline, Ketoprofen and propanolol hydrochloride as therapeutically active agents as well as many other therapeutic agents, e.g. anti-inflammatory substances indometacine, ibuprofen), coronary and cerebral vasodilators, peripheral vasodilators, anti-infectives, psychotropics and antimanics (lithium carbonate), antihistamines and anti-ulcus substances, laxatives, anti-arrhythmics and anti-hypertensive drugs, diuretics and drugs used in the treatment of migraine (dihydroergotamine mesylate) and many others.

It was found that sustained release tablets can be obtained by compressing high viscosity grade, i.e. high molecular weight hydroxypropylmethylcellulose (Methocel E4M, 4 Pa.s (4000 cps), Premium), microcrystalline cellulose, glycerol ditripalmitostearate and other conventional pharmaceutically acceptable adjuvants, and the therapeutic agent in definite proportions. When the tablet is brought in contact with water or digestive fluids, a certain percentage of the therapeutic agent is rapidly released from the preparation into the solvent. The hydratation and the swelling of cellulose take place on the contact surface of the tablet with water and a gel barrier is formed. The rest of the therapeutic agent is then released more slowly, depending on the diffusion rate across the gel barrier and/or on its attrition.

The method for manufacturing sustained release tablets is based either on direct tableting or on previous dry or wet preparation of granules, which comprises thoroughly blending the hydroxypropylmethylcellulose carrier with the therapeutic agent in powdery or granular form, with glycerol ditripalmitostearate and microcrystalline cellulose and the remaining conventional adjuvants used in the tablet manufacture, e.g. magnesium stearate, lactose, starch, i.e. binding, filling and swelling agents etc. The ingredients are compressed in conventional tableting machines to give products of desired shape, weight, hardness and low friability, thus providing for the desired prolonged release of the therapeutic agent within a period of up to 12 hours, depending on the shape and the hardness of the tablets and particularly on the carrier. Thus it is possible to produce long-acting ot sustained release tablets in a relatively simple and economical manner.

The following illustrative Examples are not to be considered limitative.

Example 1

Sustained release 350 mg aminophylline tablets (retard tablets) containing 27.8 % Methocel E4M, 4 Pa.s (4000 cps), Premium, are prepared from untreated Methocel E4M.

The 350 mg aminophylline tablets are prepared from the following ingredients:

| Ingredients | mg/tablet | % |
|---|---|---|
| Aminophylline anhydrous | 350.0 mg | 54.68 |
| Microcrystalline cellulose (Avicel) | 74.0 mg | 11.56 |
| Aerosil 200 | 6.5 mg | 1.01 |
| Precirol Ato 5 Gattefossé | 18.0 mg | 2.81 |
| Magnesium stearate | 5.5 mg | 0.85 |
| Dye FD & C 5 Yellow Al. lake | 8.0 mg | 1.25 |
| Methocel E4M, Premium | ad 640.0 mg | 27.8 |

Note:

Precirol Ato 5 (Gattefossé) is the commercial designation for glycerol ditripalmitostearate. Methocel E4M Premium is the designation of hydroxypropylmethylcellulose 4 Pa.s (4000 cps), Aerosil 200 is the designation for high purity $SiO_2$ (see H.P. Fiedler Lexikon der Hilfsstoffe fur Pharmazie, Kosmetik und angrenzende Gebiete, 2nd Edition 1981, Editio Cantor, Aulendorf, West Germany). The dye FD & C 5 Yellow Al. lake (Capsugel AG, Basel) is on the tartrazine basis.

The anhydrous aminophylline, Methocel E4M, the dye, a part of Precirol, Aerosil and magnesium stearate are passed through an appropriate sieve and after the mixing the mixture is shaped into briquettes The briquettes are ground to the desired granulation. To the granulate the remaining microcrystalline cellulose (Avicel), Precirol and magnesium stearate are added and mixed. The thus obtained granulate is compressed to tablets of desired shape, weight and hardness, whereby an adequate release of the therapeutic agent is achieved.

Test Results

Stability

The results of the analyses of accelerated and current stability tests demonstrate that the solid drug unit dosage form of sustained release 350 mg aminophylline tablets on the hydroxypropylmethylcellulose, type Methocel E4M Premium, carrier base material meets the requirements with respect to both the therapeutic agent release from the tablet and the chemical and physical stability over the whole storage time, as it is evident from the following tables with test results.

Table 1

| Storage time (months) | + Storage conditions | | | | + + Storage conditions | | | |
|---|---|---|---|---|---|---|---|---|
| | 20±5°C | 40±1°C | 50±1°C | 25°C 80% rel. humidity | 20±5°C | 40±1°C | 50±1°C | 25°C 80% rel. humidity |
| 0 | 100.0% | - | - | - | 100.0% | - | - | - |
| 3 | 99.5% | 99.2% | 99.7% | - | 100.0% | 96.5% | 95.0% | - |
| 6 | 99.9% | 99.7% | 99.2% | 96.1% | 99.5% | 95.3% | 93.8% | 95.9% |
| 12 | 101.1% | 98.9% | 97.5% | - | 98.1% | 91.0% | 86.7% | - |
| 24 | 98.5% | - | - | 96.7% | 96.4% | - | - | 96.2% |
| 37 | 97.8% | - | - | 95.8% | 96.7% | - | - | 95.8% |

+ Content: anhydrous theophylline; declared content = 300.0 mg (100 %)
+ + Content: ethylene diamine; initial content = 52.22 mg (100 %)
Note: The results are expressed as a mean value of four determinations.

Hardness (N)

Apparatus: Erweka [BT], producer Erweka-Apparatebau GmbH, Heuenstamm, Kr. Offenbach/Main, West Germany

Table 2

| Storage time (months) | Storage conditions 20 ± 5°C |
|---|---|
| 0 | 53.9 - 78.4 N |
| 3 | 53.9 - 78.4 N |
| 6 | 58.8 - 78.4 N |
| 12 | 53.9 - 88.2 N |
| 37 | 44.1 - 78.4 N |

The results are given for 10 tablets from minimum to maximum hardness.

Dissolution (release rate)

350 mg aminophylline tablets containing 300 mg of the therapeutic agent theophylline.
Storage conditions: blister - room temperature.
Apparatus: apparatus 3 (USP XX).
Medium: artificial gastric and intestinal fluids, 600 ml.
Temperature: 37°C.
Quantitative analysis: UV spectrophotometry, 275 nm.
Requirement: after 1 hour: 10-30 %
after 3.5 hours: 45-75 %
after 7 hours: 80 %

Table 3

| Time (hrs) | % of released theophylline | | |
|---|---|---|---|
| | analysis 1 | analysis 2 | analysis 3 |
| 1 | 15.7% | 15.5% | 15.2% |
| 3.5 | 58.6% | 54.3% | 52.0% |
| 7 | 89.0% | 85.1% | 84.0% |

The foregoing tables demonstrate that even after prolonged storage at elevated temperatures sustained release aminophylline tablets remain practically unaltered, which assures a shelf life of 3 years at a temperature up to 25°C.

In vitro release rate of theophylline from sustained release 350 mg aminophylline tablets (A) of the aforesaid composition and from the commercial preparation sustained release 350 mg Phyllocontin$^R$ forte tablets (P).
Apparatus 3 (USP XX).
Medium: artificial gastric and intestinal fluids, free of enzymes (USP XXI), 600 ml.
Temperature: 37°C.
Quantitative analysis: UV spectrophotometry, 275 nm.

| Time (h) | % of released theophylline ($\overline{X}$, n = 6) | |
|---|---|---|
| | A | P |
| 1 | 22.1 | 17.9 |
| 2 | 35.4 | 32.6 |
| 3.5 | 58.5 | 59.4 |
| 5 | 75.0 | 79.7 |
| 7 | 85.9 | 93.4 |

| Sustained release 350 mg Aminophylline tablets (A) | | | | |
|---|---|---|---|---|
| | $C_{max}$ ($\mu$g/ml) | $t_{max}$ (h) | $AUC^{0-24}$ ($\mu$g.h/ml) | $AUC^{0-\infty}$ ($\mu$g.h/ml) |
| mean | 4.925 | 4.4 | 73.42 | 116.09 |
| range | 3.536-6.735 | 3-6 | 49.57-103.50 | 72.21-164.89 |
| No. of s. | 10 | 10 | 10 | 10 |
| Note: | | | | |
| $C_{max}$ = maximal concentration of therapeutic agent in blood | | | | |
| $t_{max}$ = time to maximal concentration of therapeutic agent in blood | | | | |
| AUC = area under the plasma concentration of therapeutic agent curve | | | | |
| No. of s. = number of subjects | | | | |

| Sustained release 350 mg Phyllocontin® forte tablets (P) | | | | |
|---|---|---|---|---|
| | $C_{max}$ ($\mu$g/ml) | $t_{max}$ (h) | $AUC^{0-24}$ ($\mu$g.h/ml) | $AUC^{0-\infty}$ ($\mu$g.h/ml) |
| mean<br>range<br>No. of s. | 4.301<br>3.076-6.559<br>10 | 5.3<br>2-8<br>10 | 68.75<br>41.58-122.70<br>10 | 100.64<br>55.19- 187.52<br>10 |
| Note:<br>$C_{max}$ = maximal concentration of therapeutic agent in blood<br>$t_{max}$ = time to maximal concentration of therapeutic agent in blood<br>AUC = area under the plasma concentration of therapeutic agent curve<br>No. of s. = number of subjects | | | | |

The comparison preparation for sustained release 350 mg aminophylline tablets was the commercial preparation sustained release 350 mg Phyllocontin[R] forte tablets. The plasma concentration curve has a form suitable for a sustained release formulation. The pharmacokinetic parameters do not differ statistically significantly.

Example 2

Sustained release 160 mg propranolol hydrochloride tablets containing 36.2 % Methocel E4M Premium 4 Pa.s (4000 cps), are prepared from untreated Methocel E4M. The tablets are prepared from the following ingredients:

| Ingredients | mg/tablet | % |
|---|---|---|
| Propanolol hydrochloride | 160 | 46.38 |
| Methocel E4M, 4 Pa.s (4000 cps), Premium | 125 | 36.23 |
| microcrystalline cellulose | 45.2 | 13.10 |
| Precirol ATO 5 | 9.9 | 2.87 |
| Aerosil 200 | 3.3 | 0.96 |
| magnesium stearate | 1.6 | 0.46 |

Sustained release propranolol tablets were prepared by briquetting the ingredients, followed by domminuting the briquettes, sieving throuhg an appropriate size sieve and forming a granulate.

The resulting granulate is homogenously mixed under the addition of an appropriate lubricant and again passed through a sieve.

The thus obtained granulate is tableted to tablets of desired shape, weight and hardness, whereby an appropriate release of the therapeutic agent is achieved.

Test results

Stability

Table 1: Propranolol hydrochloride content

Initial content: 160 mg propranolol hydrochloride
per tablet = 100.0 %

| Storage time (days) | Storage conditions | | | | | |
|---|---|---|---|---|---|---|
| | $4\pm1^{\circ}C$ | $20\pm5^{\circ}C$ | $30\pm1^{\circ}C$ | $40\pm1^{\circ}C$ | $50\pm1^{\circ}C$ | 80 % humidity ($25^{\circ}C$) |
| 126 | 99.1% | 100.6% | 99.8% | 100.0% | 99.9% | 99.7% |
| 220 | 100.7% | 99.0% | 100.7% | 100.3% | 100.8% | 100.2% |
| 380 | 99.5% | 100.0% | 99.3% | 99.0% | 100.0% | 99.6% |

Table 2: Hardness of the tablets (N)

Apparatus: Erweka BT

Initial value: 39.2 - 68.6 N

| Storage time (days) | Storage conditions | | |
|---|---|---|---|
| | $4^{\circ} \pm 1^{\circ}C$ | $20^{\circ} \pm 5^{\circ}C$ | $30^{\circ} \pm 1^{\circ}C$ |
| 126 | 58.8 - 68.6 | 63.7 - 68.6 | 63.7 - 73.5 |
| 220 | 49.0 - 63.7 | 44.1 - 73.5 | 58.8 - 73.5 |
| 380 | 73.5 - 88.2 | 73.5 - 88.2 | 78.4 - 88.2 |

Table 3: Dissolution test (release rate)

Propranolol hydrochloride: 160 mg tablets

Storage conditions: blister - room temperature

Apparatus 3 (USP XX)

Medium: artificial gastric fluid and intestinal fluids free of enzymes (USP XXI), 600 ml

Temperature: $37^\circ C$

Quantitative analysis: UV spectrophotometry, 286 nm

Requirement: after 1 hour: 10-30 %

after 4 hours: 40-75 %

after 8 hours: > 75 %

| Time (hrs) | % of released propranolol hydrochloride | | | | |
|---|---|---|---|---|---|
| | analysis 1 | analysis 2 | analysis 3 | analysis 4 | analysis 5 |
| 1 | 26.8 | 22.0 | 22.5 | 22.8 | 22.5 |
| 2 | 41.4 | 35.4 | 37.0 | 37.2 | 35.9 |
| 4 | 62.9 | 56.3 | 56.5 | 57.7 | 56.0 |
| 6 | 78.2 | 73.1 | 70.6 | 74.1 | 71.8 |
| 8 | 88.8 | 83.4 | 81.1 | 83.7 | 84.0 |

On the basis of accelerated stability test results it was found that sustained release 160 mg propranolol hydrochloride tablets are stable and at at the temperature of 25 ° C a shelf life of 3 years can be assured.

In vitro release rate of propranolol hydrochloride from sustained release 160 mg tablets with aforesaid composition (P) and form the commercial preparation sustained release 160 mg Inderal[R] tablets (I).

Apparatus 3 (USP XX)

Medium: artificial gastric and intestinal fluids free of enzymes (USP XXI), 600 ml

Temperature: 37 ° C

Quantitative analysis: UV spectrophotometry, 286 nm

| Time (hrs) | % of released propranolol hydrochloride | |
|---|---|---|
| | P | I |
| 1 | 19.5 | 17.0 |
| 2 | 31.8 | 35.2 |
| 4 | 50.3 | 56.2 |
| 6 | 65.5 | 69.0 |
| 8 | 77.3 | 77.4 |

| Sustained release 160 mg propranolol hydrochloride tablets | | | | |
|---|---|---|---|---|
| | $t_{max}$ (h) | $C_{max}$ (ng/ml) | $AUC^{0-24}$ (ng.h/ml) | $AUC^{0-\infty}$ (ng.h/ml) |
| mean | 5.0 | 22.09 | 292.29 | 492.92 |
| range | 2.0-13.8 | 11.10-41.50 | 134.68-727.70 | 194.64-1695.31 |
| No. of s. | 8 | 8 | 8 | 8 |

Note:

$C_{max}$ = maximal concentration of therapeutic agent in blood

$t_{max}$ = time to maximal concentration of therapeutic agent in blood AUC = area under the plasma concentration of therapeutic agent curve No. of s. = number of subjects

| Sustained release 160 mg Inderal[R] tablets | | | | |
|---|---|---|---|---|
| | $C_{max}$ (ng/ml) | $t_{max}$ (h) | $AUC^{0-24}$ (ng.h/ml) | $AUC^{0-\infty}$ (ng.h/ml) |
| mean | 16.64 | 6.75 | 20.60 | 389.71 |
| range | 4.30-25.40 | 5-12 | 73.10-383.23 | 163.74-1376.28 |
| No. of s. | 8 | 8 | 8 | 8 |

Note:

$C_{max}$ = maximal concentration of therapeutic agent in blood

$t_{max}$ = time to maximal concentration of therapeutic agent in blood AUC = area under the plasma concentration of therapeutic agent curve No. of s. = number of subjects

The comparison preparation for sustained release 160 mg propranolol hydrochloride tablets were sustained release 160 mg Inderal[R] tablets. The above results demonstrate the superior bioavailability of sustained release propranolol tablets (approximately 25 %, as calculated from $AUG^{0-\infty}$).The plasma concentration diagrams are suitable for a sustained release form.

## Claims

1. Sustained release tablets, comprising aminophylline, theophylline, ketoprofen for propranolol hydrochloride as the therapeutically active agent, hydroxypropyl methylcellulose having a methoxyl content of 28 to 30 weight-%, a hydroxypropoxyl content of 7.5 to 12 weight-% and an average molecular weight of at least 50000 in a proportion of 20 to 40 weight-% of the tablet as the carrier base material, from 10 to 20 weight-% of microcrystalline cellulose, other conventional pharmaceutically acceptable adjuvants, and additionally containing glycerol ditripalmitostearate in an amount up to 10 weight-%.

2. A process for the manufacture of sustained release tablets as claimed in claim 1, characterized in that the mixture of a therapeutic agent and a carrier base material comprising hydroxypropyl methylcellulose having a methoxyl content of 28 to 30 weight-%, a hydroxypropoxyl content of 7.5 to 12 weight-% and an average molecular weight of at least 50000 in a proportion of 20 to 40 weight-% of the tablet, from 10 to 20 weight-% of microcrystalline cellulose, other conventional pharmaceutically acceptable adjuvants, and additionally, containing glycerol ditripalminostearate in an amount up to 10 weight-%, is compressed and shaped.

## Patentansprüche

1. Tabletten mit verzögerter Freisetzung, enthaltend Aminophyllin, Theophyllin, Ketoprofen oder Propranolol-Hydrochlorid als therapeutischen Wirkstoff, Hydroxypropylmethylcellulose mit einem Methoxylgehalt von 28 bis 30 Gew.%, einem Hydroxypropoxylgehalt von 7.5 bis 12 Gew.% und mit einem mittleren Molekulargewicht von mindestens 50000 in einem Anteil von 20 bis 40 Gew.% der Tablette als Trägergrundmaterial, 10 bis 20 Gew.% mikrokristallinische Cellulose, andere pharmazeutisch annehmbare Hilfsmittel und zusätzlich Glycerinditripalmitostearat in einer Menge bis zu 10 Gew.%.

**2.** Verfahren zur Herstellung von Tabletten mit verzögerter Freisetzung nach Anspruch 1, dadurch gekennzeichnet, dass man das Gemisch aus einem therapeutischen Wirkstoff und einem Trägergrundmaterial enthaltend Hydroxypropylmethylcellulose mit einem Methoxylgehalt von 28 bis 30 Gew.%, einem Hydroxypropoxylgehalt von 7.5 bis 12 Gew.% und mit einem mittleren Molekulargewicht von mindestens 50000 in einem Anteil von 20 bis 40 Gew.% der Tablette, 10 bis 20 Gew.% mikrokristallinische Cellulose, andere pharmazeutisch annehmbare Hilfsmittel und zusätzlich Glycerinditripalmitostearat in einer Menge bis zu 10 Gew.%, verpresst und formt.

**Revendications**

**1.** Comprimes à libération soutenue de la substance médicamenteuse, comprenant de l'aminophylline, de la théophylline, du cétoprofène, ou du chlorhydrate de propranolol, à titre d'agent thérapeutiquement actif, de l'hydroxypropylméthylcellulose possédant une teneur en radicaux méthoxyle de 28 à 30% en poids, une teneur en radicaux hydroxypropoxyle de 7,5 à 12% en poids et un poids moléculaire moyen d'au moins 50.000, en une proportion de 20 à 40% en poids du comprimé, à titre de matières servant de véhicule ou d'excipient de base, de 10 à 20% en poids de cellulose microcristalline, d'autres adjuvants pharmaceutiquement acceptables classiques et contenant, de surcroît, du ditripalmitostéarate de glycérol en une proportion allant jusqu'à 10% en poids.

**2.** Procédé de fabrication de comprimés à libération soutenue de la substance médicamenteuse suivant la revendication 1, caractérisé en ce que l'on comprime et façonne ou moule le mélange d'un agent thérapeutique et d'une matière servant de véhicule ou excipient de base, constituées d'hydroxypropylméthylcellulose possédant une teneur en radicaux méthoxyle de 28 à 30% en poids, une teneur en radicaux hydroxypropoxyle de 7,5 à 12% en poids et un poids moléculaire moyen d'au moins 50.000, en une proportion de 20 à 40% en poids du comprimé, de 10 à 20% en poids de cellulose microcristalline, d'autres adjuvants pharmaceutiquement acceptables classiques et contenant, de surcroît, du ditripalmitostéarate de glycérol en une proportion allant jusqu'à 10% en poids.